# EUROPÄISCHE PATENTSCHRIFT

(11) **EP 3 674 680 B1**
(45) Veröffentlichungstag und Bekanntmachung des Hinweises auf die Patenterteilung: **22.09.2021**
(21) Anmeldenummer: 19217073.6
(22) Anmeldetag: 05.02.2015
(51) Int. Cl.: G01N 1/20, G01N 33/18

(54) **VERFAHREN ZUM BETREIBEN EINES MOBILEN HANDGERÄTS ZUM MESSEN VON WASSERPARAMETERN**
METHOD OF OPERATING A MOBILE HANDSET FOR MEASURING PARAMETERS OF WATER
PROCÉDÉ DE FONCTIONNEMENT D'UN DISPOSITIF PORTATIF MOBILE POUR MESURER DES PARAMÈTRES D'EAU

(30) Priorität: 11.02.2014 DE 102014001716
(43) Veröffentlichungstag der Anmeldung: 01.07.2020
(62) Teilanmeldung aus: 15000333.3
(73) Patentinhaber: Erwin Quarder Systemtechnik GmbH, 32339 Espelkamp (DE)
(72) Erfinder: Kottkamp, Eike, 32312 Lübbecke (DE)
(74) Vertreter: Aulich, Martin

(56) Entgegenhaltungen:
- WO-A1-2005/052573
- WO-A1-2013/158515
- DE-A1-102012 009 076
- US-A1- 2002 130 069
- US-A1- 2006 020 427

## Beschreibung

Die vorliegende Erfindung betrifft ein Verfahren zum Betreiben eines mobilen Handgeräts zur Messung von physikalischen und/oder chemischen und/oder biologischen Parametern von aus einer Wasserentnahmestelle fließendem Wasser, insbesondere Trinkwasser.

Die Trinkwasserverordnung in Deutschland fordert sowohl für den privaten als auch für den gewerblichen Bereich jährlich durchzuführende Prüfungen der Trinkwasserqualität. Solche Prüfungen umfassen insbesondere die Messung diverser Wasserparameter. Es besteht das Bedürfnis, solche Messungen möglichst zuverlässig, schnell und kostengünstig durchführen zu können.

In der WO 2005/052573 A1 ist ein an einen Hydranten angeschlossenes Gerät offenbart, mit dem bestimmte Wasserparameter gemessen werden können. Die Prüfung der Wasserparameter erfolgt automatisiert über mehrere Tage oder Wochen, ohne dass ein Bediener eingreifen muss. Das Gerät verfügt unter anderem über einen Zulauf, durch den Wasser durch das Gerät fließen kann, und einen Ablauf, durch den das Wasser nach Durchfluss durch das Gerät aus diesem abfließen kann. Das Gerät verfügt weiter über eine Messeinrichtung, mit der Wasserdruck des durchfließenden Wassers gemessen werden kann. Über eine hinterlegte Tabelle kann aus dem Wasserdruck die Zeit bestimmt werden, nach der eine bestimmte, als ausreichend beschriebene Menge Wasser durch das Gerät geflossen ist. Der Durchfluss wird dann mittels einer Steuereinheit, die ein entsprechendes Ventil betätigt, nach Ablauf dieser Zeit gestoppt und die Temperatur des Wassers wird gemessen.

In den Druckschriften US 2006/020427 A1, US 2002/130069 A1, DE 10 2012 009 076 A1 und WO2013/158515 A1 sind weitere Varianten von Messeinrichtungen zur Messung von

Wasserparametern offenbart. In der US2006/020427 A1 wird eine tragbare, in einer Hand haltbare, Sensoreinheit an ein Trinkwasserverteilungssystem angeschlossen. Einer der Sensoren ist ein Temperatursensor.

Hiervon ausgehend liegt der Erfindung die Aufgabe zugrunde, für die eingangs genannten Messungen ein geeignetes Verfahren zum Betreiben des eingangs genannten mobilen Handgeräts anzugeben.

Diese Aufgabe wird gelöst durch ein Verfahren zum Betreiben desselben mit den Merkmalen des Anspruchs 1.

Die Erfindung schlägt demnach vor, für die Messung von physikalischen und/oder chemischen und/oder biologischen Wasserparametern von insbesondere Trinkwasser ein mobiles Handgerät einzusetzen und dieses mit dem Verfahren gemäß Anspruch 1 zu betreiben, wobei das Handgerät über mehrere Messeinrichtungen verfügt, mit denen unterschiedliche Wasserparameter gemessen werden können. Im Regelfall umfasst dabei die Wasserentnahmestelle, jedenfalls in Privathaushalten, einen sogenannten Wasserhahn, aus dem das Trinkwasser entnommen werden kann. Die Erfindung sieht vor, das mobile Handgerät zeitweise, nämlich mindestens für die Dauer der Messungen, an den Wasserauslass der zu prüfenden Wasserentnahmestelle anzuschließen, insbesondere an den Wasserauslass eines bzw. des Wasserhahns der Wasserentnahmestelle.

Das Handgerät kann erfindungsgemäß über einen Zulauf verfügen, durch den das Wasser aus dem Wasserauslass der Wassersentnahmestelle in das Handgerät fließen bzw. strömen kann. Weiter kann es über einen Ablauf verfügen, durch den das Wasser nach dem Durchfluss durch das Handgerät wieder aus diesem herausfließen kann, insbesondere nachdem es entlang der Messeinrichtungen geführt wurde. Das aus dem Ablauf des Handgerätes herausfließende Wasser kann dann beispielsweise in Richtung eines unter dem Handgerät angeordneten Waschbeckens oder dergleichen abfließen.

Weiter weist das Handgerät ein Ventil auf, mit dem der Durchfluss des Wassers durch das Handgerät bzw. das Abfließen des Wassers aus dem Ablauf gesperrt bzw. gestoppt werden kann.

Jedenfalls eine der Messeinrichtungen ist erfindungsgemäß ein Wassermengenzähler. Dieser ist wichtig, um für die spätere Entnahme von Wasserproben mithilfe von separaten Probengefäßen an unterschiedlichen Wasserentnahmestellen definierte, jeweils vergleichbare Ausgangsbedingungen schaffen zu können, wie dies später noch näher erläutert wird.

Der Wassermengenzähler ist dabei so ausgebildet, dass mit ihm die Menge (bzw. das Volumen) des Wassers bestimmt werden kann, die seit dem Beginn der entsprechenden Wassermengenmessung durch das an den Wasserauslass angeschlossene Handgerät bzw. entlang des Wassermengenzählers fließt.

Zweckmäßigerweise wird das Handgerät für die nachfolgende Wassermengenmessung zunächst an den Wasserauslass angeschlossen, dann ggf. das Ventil der Wasserentnahmestelle - regelmäßig ein handelsüblicher Wasserhahn - geöffnet und die Wassermengenmessung anschließend bevorzugt durch einen Bediener des Handgeräts in nachfolgend noch näher beschriebener Weise gestartet. Dies in der Regel zum Zeitpunkt der Öffnung des Wasserhahns oder zu einem beliebigen späteren Zeitpunkt.

Das Handgerät kann eine elektronische Steuereinheit aufweisen, mit der das Absperrventil des Handgeräts betätigt bzw. geöffnet und geschlossen werden kann. Erfindungsgemäß ist nun vorgesehen, dass die elektronische Steuereinheit des Handgeräts das Absperrventil automatisch schließt, sodass der Wasserfluss durch das Handgerät und somit letztlich auch der weitere Wasserfluss aus der Wasserentnahmestelle unterbrochen wird, wenn die Messungen des Wassermengenzählers ergeben, dass die Wassermenge, die seit Beginn der Messungen durch das Handgerät geströmt sind, eine vorbestimmte Bedingung erfüllt. Beispielsweise kann der Durchfluss des Wassers durch das Handgerät auf diese Weise gestoppt werden, wenn eine bestimmte, vordefinierte Wassermenge, beispielsweise gemessen in Litern, durch das Handgerät geströmt ist.

Der Start der oben beschriebenen Wassermengenmessung kann beispielsweise durch Betätigung einer von dem Bediener betätigbaren Eingabeeinrichtung des Handgeräts erfolgen, die mit der Steuereinheit wirkverbunden ist bzw. über die die Steuereinheit bedien- bzw. beeinflussbar ist.

So kann vorgesehen sein, dass das Absperrventil des Handgerätes bei erstmaligem Anschluss an die Wasserentnahmestelle zunächst standardmäßig geschlossen ist. Anschließend öffnet der Bediener das Ventil des Wasserauslasses bzw. den Wasserhahn. Zum Start der nachfolgenden Wassermengenmessung betätigt der Bediener die Eingabeeinrichtung, indem er etwa ein entsprechendes Betätigungsorgan der Eingabeeinrichtung betätigt, wie etwa eine bestimmte Taste etc.

Hierdurch getriggert öffnet die Steuereinheit zum einen das Absperrventil, zum anderen sendet sie der Wassermengenmesseinrichtung ein entsprechendes Startsignal, sodass diese ihre Messungen beginnt. Die Steuereinheit überwacht bzw. prüft anschließend, ob mindestens einer der Messwerte, die der Wassermengenzähler liefert, die vorgegebene Bedingung erfüllt. Sobald dies der Fall ist, schließt sie das Absperrventil.

Unmittelbar nach dem Schließen des Absperrventils oder aber nachdem mittels der oder den anderen Messeinrichtungen des Handgeräts zusätzlich eine oder mehrere weitere Messungen durchgeführt wurden, kann dann der Wasserentnahmestelle die Wasserprobe entnommen werden, beispielsweise um diese in einem Labor separat untersuchen zu können, beispielsweise auf Verkeimung, etwa durch Legionellen.

Für die eigentliche Probennahme, d.h. das Abfüllen einer bestimmten Wassermenge in ein entsprechendes Gefäß, wird das Handgerät vorteilhafterweise zunächst von dem Wasserauslass abgenommen bzw. von diesem entfernt.

Das mobile Handgerät verfügt insgesamt, wie oben bereits angedeutet, über mindestens eine, bevorzugt mehrere weitere Messeinrichtungen, die zur Analyse des Wassers sinnvoll bzw. sehr hilfreich sind.

Bevorzugt weist das Handgerät beispielsweise neben dem Wassermengenzähler eine Messeinrichtung auf zur Messung des statischen Leitungsdrucks in der Wasserleitung, an die die Wasserentnahmestelle angeschlossen ist.

Weiter kann das Handgerät einen Durchflussmesser zur Messung der pro Zeiteinheit aus der Wasserentnahmestelle fließenden Wassermenge aufweisen.

Das Handgerät verfügt über eine Temperaturmesseinrichtung, sodass die Temperatur des aus der Wasserentnahmestelle stammenden Wassers messbar ist.

Weitere Messeinrichtungen des Handgeräts können zur Messung des pH-Wertes, zur Messung der elektrischen Leitfähigkeit und/oder zur Messung von (sonstigen) chemischen oder ggf. biologischen Eigenschaften des Wassers dienen bzw. ausgebildet sein.

Gemäß einer wichtigen Weiterbildung der Erfindung ist vorgesehen, dass das Handgerät an verschiedenste Arten von Wasserauslässen von Wasserentnahmestellen, insbesondere von Wasserauslässen von Wasserhähnen derselben, angeschlossen werden kann. Vorgesehen ist zu diesem Zweck die Verwendung eines separaten Adapters, der einerseits mit dem Wasserauslass der Wasserentnahmestelle lösbar verbunden werden kann, andererseits mit dem Zulauf des Handgeräts. Der Adapter ist entsprechend sowohl an den Zulauf des Handgeräts als auch an den Wasserauslass der Wasserentnahmestelle angepasst. Bevorzugt weist der Adapter für den Anschluss desselben an den Wasserauslass einen Schnellverschluss auf, beispielsweise einen Bügelverschluss oder einen Klemmverschluss, sodass der Adapter in einfacher und vor allem schneller Art und Weise mit dem Wasserauslass verbunden werden kann.

Gemäß weiterer Ausbildung der Erfindung ist vorgesehen, für die Messungen des Handgeräts jeweils ein separates, bevorzugt steriles Wasserführungsteil vorzusehen, das in den Zulauf des Handgeräts eingebracht werden kann, bevor das Handgerät an den Wasserauslass angeschlossen wird. Bevorzugt ist das Wasserführungsteil hierzu in den Zulauf einschraubbar bzw. eingeschraubt oder allgemein kraft- und/oder formschlüssig mit dem Zulauf verbunden. Entsprechend ist der Zulauf des Handgeräts so ausgebildet, dass es ein solches separates Wasserführungsteil aufnehmen kann. Hierdurch kann eine (Rück-)Verkeimung des Wasserauslasses bzw. der Wasserentnahmestelle durch das Handgerät begrenzt oder verhindert werden.

Das Wasserführungsteil wird dabei so platziert, dass nach Verbindung des Handgeräts mit dem Wasserauslass (ggf. über den Adapter) das aus dem Wasserauslass fließende Wasser durch das Wasserführungsteil in das Innere des Handgeräts geführt wird, nämlich zu oder entlang der Messeinrichtungen. Und zwar bevorzugt, ohne dass wasserführende Flächen bzw. Teile des Wasserauslasses direkt mit den übrigen Teilen des Handgeräts in Berührung kommen können.

Bevor mit dem Handgerät nach erfolgten Messungen neue Messungen an einer anderen Wasserentnahmestelle durchgeführt werden, kann dann das Wasserführungsteil aus dem Zulauf entnommen, separat sterilisiert und anschließend wieder eingesetzt werden. Oder es kann schlicht durch ein neues, steriles Wasserführungsteil ersetzt werden. In diesem Fall wäre das Wasserführungsteil jeweils als Einweg-Produkt ausgebildet. Bei einem solchen Wasserführungsteil kann es sich beispielsweise um ein rohrartiges bzw. röhrchenartiges Teil handeln.

Hintergrund dieser Maßnahme ist, dass das Handgerät, insbesondere dessen Messeinrichtungen im Inneren, nicht oder nur sehr schwierig sterilisiert werden können.

In weiterer Ausbildung der Erfindung verfügt das Handgerät im Übrigen über eine Anzeigeeinheit, über die dem Bediener Ergebnisse von durchgeführten Messungen anzeigbar sind und/oder über eine Sendeeinheit, mit der die Ergebnisse der durchgeführten Messungen, insbesondere drahtlos, an eine entfernte Empfangseinheit gesendet werden können. Natürlich ist auch denkbar, die Ergebnisse in einem internen Speicher des Handgeräts abzuspeichern. Im Hinblick auf die Verarbeitung, Auswertung und Weiterleitung der Messergebnisse gibt es naturgemäß vielfältige Möglichkeiten.

Was weitere automatische oder teilautomatischer Steuerungs- bzw. Programmabläufe des Handgeräts betrifft, gibt es naturgemäß verschiedenste Möglichkeiten.

Bevorzugt ist unter anderem vorgesehen, dass nach dem oben erwähnten automatischen Schließvorgang des Absperrventils, wenn also die durch das Handgerät durchgeflossene Wassermenge die vorbestimmte Bedingung erfüllt hat, mittels der Messeinrichtung des Handgeräts zur Messung des statischen Wasserdrucks automatisch der statische Wasserdruck gemessen wird, der stromauf des Ventils und somit in der Wasserleitung anliegt, an die die Wasserentnahmestelle angeschlossen ist. Mit anderen Worten wird durch die Steuereinheit nach dem Schließen des Ventils automatisch die vorgenannte Messeinrichtung getriggert, sodass diese beginnt die Messungen des statischen Wasserdrucks vorzunehmen.

Ein besonderes Ablaufprogamm umfasst folgende Maßnahmen:
Beispielsweise kann im ersten Schritt die oben erwähnte Wassermengenmessung in einem Kaltwasserfluss erfolgen, indem das Ventil der Wasserentnahmestelle bzw. der Wasserhahn von einem Bediener so eingestellt wird, dass aus dem Wasserauslass entsprechend zunächst kaltes Wasser strömt, bis die Steuereinheit das Absperrventil aufgrund der von der Wasserdurchflussmenge erfüllten Bedingung in der oben beschriebenen Weise schließt.

Bevorzugt nach diesem Schließvorgang des Absperrventils, insbesondere nachdem, wie oben beschrieben, die (Kalt-)Wasserprobe entnommen wurde und ggf. automatisch auch der statische Kaltwasserleitungsdruck und ggf. auch die Kaltwassertemperatur und ggf. zudem weitere Wasserparameter des Kaltwassers gemessen wurden, stellt der Bediener den Wasserhahn auf die Stellung "Warmwasser". Insofern das Handgerät zuvor von dem Wasserauslass abgenommen wurde, schließt er es wieder an, insbesondere mithilfe des Adapters mit Schnellverschluss.

Der Bediener betätigt dann die Eingabeeinrichtung, wodurch das Absperrventil, getriggert durch die mit der Eingabeeinrichtung zusammenwirkende Steuereinheit, wieder geöffnet wird, sodass das Warmwasser durch das Handgerät hindurch läuft und schließlich aus dem Ablauf des Handgeräts herausläuft.

Getriggert durch die Steuereinheit wird dann mittels der Temperaturmesseinrichtung des Handgeräts automatisch mehrfach die Temperatur des aus der Wasserentnahmestelle fließenden Warmwassers gemessen, insbesondere kontinuierlich.

Das Absperrventil wird dann, ebenfalls automatisch getriggert durch die Steuereinheit, (erneut) geschlossen, wenn die Temperaturmessungen ergeben, dass die Wassertemperatur des Warmwassers einer vorbestimmten Bedingung entspricht, insbesondere in einer vorbestimmten Weise stabil ist. Als stabil kann sie von der Steuereinheit beispielsweise dann bewertet werden, wenn sich die gemessenen Temperaturwerte über einen definierten Messzeitraum nicht mehr oder nur innerhalb eines Toleranzbereichs ändern.

Weiter kann vorgesehen sein, dass nach dem vorgenannten erneuten Schließvorgang des Ventils der statische Wasserdruck, diesmal derjenige des Warmwassers, mittels der Messeinrichtung des Handgeräts zur Messung des statischen Wasserdrucks erneut automatisch, d.h. getriggert durch die Steuereinheit, gemessen wird. Also derjenige statische Druck, der stromauf des Absperrventils und somit diesmal entsprechend in der Warmwasserleitung anliegt, an die die Wasserentnahmestelle angeschlossen ist. Auch kann vorgesehen sein, das automatisch ggf. zudem in der oben beschriebenen Weise weitere Wasserparameter des Warmwassers gemessen werden.

Nach Abschluss der jeweiligen oder nach Abschluss mehrerer oder sämtlicher Messungen kann/können auf der Anzeigeeinheit des Handgeräts automatisch ein oder mehrere Ergebnisse der durchgeführten Messungen angezeigt werden. Alternativ oder zusätzlich kann vorgesehen sein, dass automatisch ein oder mehrere Messergebnisse der durchgeführten Messungen an eine entfernte Empfangseinheit gesendet werden.

Im Übrigen kann vorgesehen sein, dass der Bediener durch den Mess- bzw. im Programmablauf des Handgeräts interaktiv mittels entsprechender, auf der Anzeigeeinheit des Handgeräts erscheinender Anweisungen geführt wird. Beispielsweise, indem er jeweils dazu aufgefordert wird, ein oder mehrere bestimmte Betätigungsorgane der Eingabeeinrichtung zu betätigen, um etwa das Handgerät bzw. die Steuereinheit dazu zu bringen, dass dieses die Wassermengenmessung im Kaltwasserfluss startet oder etwa die Temperaturmessungen des Warmwassers.

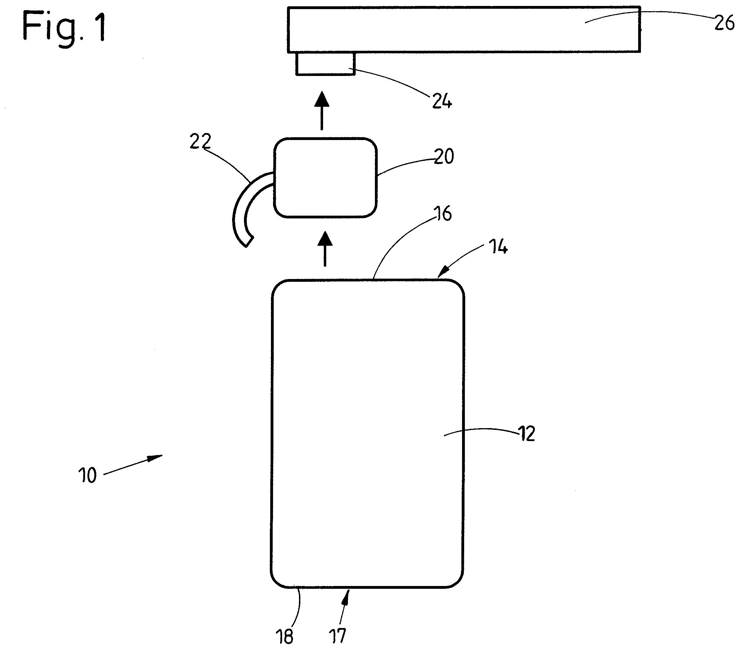

In den beigefügten Prinzipskizzen der Fig. 1-2, ist nur beispielhaft ein Handgerät 10 gezeigt, das in der vorstehend beschriebenen Weise arbeiten kann.

Das dargestellte Handgerät 10 verfügt über ein von einem Bediener gut in einer Hand zu haltendes Gehäuse 12 mit einem nicht dargestellten Zulauf im oberen Gehäusebereich 14, der insbesondere an der Gehäuseoberseite 16 angeordnet ist, sowie einem ebenfalls nicht zu erkennenden Ablauf im Bodenbereich 17, der insbesondere an dem Gehäuseboden 18 angeordnet ist.

Der nicht dargestellte Zulauf des Handgeräts 10 kann mit einem separaten, ebenfalls einen geeigneten Zulauf und Ablauf aufweisenden Adapter 20 verbunden werden.

Bevorzugt verfügt der Adapter 20 über mindestens einen mit einem Betätigungsorgan 22 betätigbaren Schnellverschluss. Im vorliegenden Ausführungsbeispiel verfügt er über zwei Schnellverschlüsse.

Der Adapter 20, nämlich dessen Zulauf, kann mittels des ersten Schnellverschlusses beispielsweise mit dem Wasserauslass 24 eines Wasserhahns 26 einer Wasserentnahmestelle, beispielsweise einer Trinkwasserentnahmestelle in einem Privathaus, lösbar verbunden werden.

Mittels eines zweiten Schnellverschlusses kann der Ablauf des Adapters 20 mit dem Zulauf des Handgeräts 10 verbunden werden.

Die beiden Schnellverschlüsse können jeweils beispielsweise eine Klemmverbindung zwischen Wasserauslass 24 und Adapterzulauf bzw. Adapterablauf und Handgerätzulauf herstellen.

Die Messeinrichtungen, die das Handgerät 10 aufweist, um die eingangs beschriebenen Messungen durchführen zu können, nämlich der Wassermengenzähler und die Temperaturmesseinrichtung, aber auch sämtliche anderen Messeinrichtungen, sind nicht gezeigt. Sie sind im Stand der Technik sämtlich bekannt und werden daher auch nicht separat beschrieben. Erfindungsgemäß werden diese Messeinrichtungen in das Handgerät 10 in geeigneter Weise integriert.

Im Inneren weist das Handgerät 10 naturgemäß ein oder mehrere Kanäle auf, entlang dem/der das zu analysierende, aus dem Wasserhahn 26 fließende Wasser der Wasserentnahmestelle geführt wird. Die Messeinrichtungen des Handgeräts 10 sind entsprechend so im Bereich bzw. in der Nähe des/der Kanäle positioniert, dass sie die entsprechenden Messungen ausführen können.

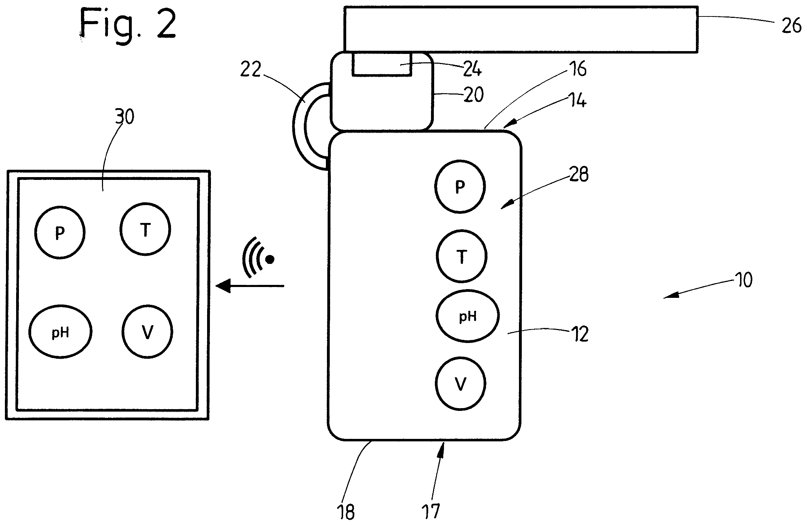

In Fig. 2 ist beispielhaft eine Anzeige- und Eingabeeinrichtung 28 gezeigt, auf der dem Bediener die Messergebnisse angezeigt werden. Die Messergebnisse können beispielsweise mit einer Sendeeinheit des Handgeräts 10 zum Senden von elektromagnetischen Wellen, etwa einem Funkmodul, drahtlos an eine entfernte, entsprechende Empfangseinheit 30 zum Empfangen elektromagnetischer Wellen gesendet werden. Die Anzeige- und Eingabeeinrichtung 28 ist als Touchscreen ausgebildet, sodass hierüber von einem Bediener auch entsprechende Eingaben getätigt werden können, die beispielsweise die oben beschriebenen Steuerungsabläufe triggern.

### Bezugszeichenliste:

- 10: Handgerät
- 12: Gehäuse
- 14: Gehäusebereich
- 16: Gehäuseoberseite
- 17: Bodenbereich
- 18: Gehäuseboden
- 20: Adapter
- 22: Betätigungsorgan
- 24: Wasserauslass
- 26: Wasserhahn
- 28: Eingabeeinrichtung
- 30: Empfangseinheit

## Patentansprüche

1. Verfahren zum Betreiben eines mobilen Handgeräts (10) zum Messen von Wasserparametern von aus einer Wasserentnahmestelle fließendem Wasser, insbesondere Trinkwasser, das für die Durchführung von Messungen von physikalischen und/oder chemischen und/oder biologischen Wasserparametern an einen Wasserauslass (24) einer Wasserentnahmestelle angeschlossen ist, wobei das mobile Handgerät (10) über mehrere Messeinrichtungen zur Messung der Wasserparameter verfügt, wobei mit einer Messeinrichtung, nämlich mit einem Wassermengenzähler, im Rahmen einer Wassermengenmessung die Menge bzw. das Volumen von seit Beginn der Wassermengenmessung aus der Wasserentnahmestelle durch das mobile Handgerät (10) fließenden Wassers gemessen wird, wobei ein Ventil des mobilen Handgeräts (10), mit dem der Durchfluss des Wassers durch das mobile Handgerät (10) gesperrt werden kann, automatisch geschlossen wird, wenn die Wassermengenmessung ergibt, dass die Wassermenge, die seit Beginn der Messung durch das mobile Handgerät (10) geflossen ist, einer vorbestimmten Bedingung entspricht, wobei mittels einer Messeinrichtung des Handgeräts (10) zur Messung der Wassertemperatur nach dem Schließvorgang des Ventils automatisch die Temperatur des aus der Wasserentnahmestelle stammenden Wassers gemessen wird, wobei nach dem Schließvorgang des Ventils und nachdem aus der Wasserentnahmestelle temperaturerhöhtes Wasser fließt, das Ventil wieder geöffnet wird und die Temperatur des aus der Wasserentnahmestelle fließenden Wassers mittels der Messeinrichtung des Handgeräts (10) zur Messung der Wassertemperatur mehrfach gemessen wird, insbesondere kontinuierlich, und wobei das Ventil automatisch erneut geschlossen wird, wenn die Messungen ergeben, dass die Wassertemperatur vorbestimmten Kriterien entspricht, insbesondere in einer vorbestimmten Weise stabil ist.

2. Verfahren gemäß Anspruch 1, **dadurch gekennzeichnet, dass** nach dem Schließvorgang des Ventils mittels einer Messeinrichtung des mobilen Handgeräts (10) zur Messung des statischen Wasserdrucks automatisch der statische Wasserdruck gemessen wird, der stromauf des Ventils und somit in der Wasserleitung anliegt, an die die Wasserentnahmestelle angeschlossen ist.

3. Verfahren gemäß einem oder mehreren der weiteren Ansprüche, **dadurch gekennzeichnet, dass** das Öffnen des Ventils nach dem Schließvorgang des Ventils und nachdem aus der Wasserentnahmestelle temperaturerhöhtes Wasser fließt durch eine Betätigung einer Eingabeeinrichtung (28) des mobilen Handgeräts (10) durch einen Bediener ausgelöst wird, wobei die Eingabeeinrichtung mit einer das Ventil steuernden elektronischen Steuereinrichtung wirkverbunden ist.

4. Verfahren gemäß Anspruch 3 wenn abhängig von Anspruch 2, **dadurch gekennzeichnet, dass** nach dem erneuten Schließvorgang des Ventils mittels der Messeinrichtung des mobilen Handgeräts (10) zur Messung des statischen Wasserdrucks automatisch erneut der statische Wasserdruck gemessen wird, der stromauf des Ventils und somit in der Wasserleitung anliegt, an die die Wasserentnahmestelle angeschlossen ist.

5. Verfahren gemäß einem oder mehreren der vorherigen Ansprüche, **dadurch gekennzeichnet, dass** nach Abschluss der Messungen, die mit dem mobilen Handgerät (10) durchgeführt werden, auf einer Anzeigeeinheit (28) des mobilen Handgeräts (10) automatisch eine oder mehrere Ergebnisse der Messungen angezeigt werden, und/oder dass automatisch eine oder mehrere Ergebnisse der durchgeführten Messungen an eine entfernte Empfangseinheit gesendet werden.

## Claims

1. Method for operating a mobile handheld device (10) for measuring water parameters of water flowing from a water intake point, in particular drinking water, which mobile handheld device is connected to a water outlet (24) of a water intake point in order to measure physical and/or chemical and/or biological water parameters, wherein the mobile handheld device (10) has a plurality of measuring devices for measuring the water parameters, wherein, as part of a water quantity measurement, one measuring device, namely a water meter, is used to measure the quantity or volume of water flowing through the mobile handheld device (10) from the water intake point since the beginning of the water quantity measurement, wherein a valve of the mobile handheld device (10) which can be used to block the flow of water through the mobile handheld device (10) is automatically closed if the water quantity measurement reveals that the quantity of water which has flowed through the mobile handheld device (10) since the beginning of the measurement corresponds to a predetermined condition, wherein a measuring device of the handheld device (10) for measuring the water temperature is used to automatically measure the temperature of the water coming from the water intake point after the closing operation of the valve, wherein, after the closing operation of the valve and after water of an elevated temperature flows from the water intake point, the valve is opened again and the temperature of the water flowing from the water intake point is measured repeatedly, in particular continuously, by means of the measuring device of the handheld device (10) for measuring the water temperature, and wherein the valve is automatically closed again if the measurements reveal that the water temperature corresponds to predetermined criteria, in particular is stable in a predetermined manner.

2. Method according to Claim 1, **characterized in that**, after the closing operation of the valve, a measuring device of the mobile handheld device (10) for measuring the static water pressure is used to automatically measure the static water pressure which is upstream of the valve and is therefore in the water line to which the water intake point is connected.

3. Method according to one or more of the further claims, **characterized in that** the opening of the valve after the closing operation of the valve and after water of an elevated temperature flows from the water intake point is initiated by an operator actuating an input device (28) of the mobile handheld device (10), wherein the input device is operatively connected to an electronic control device controlling the valve.

4. Method according to Claim 3, if dependent on Claim 2, **characterized in that**, after the renewed closing operation of the valve, the measuring device of the mobile handheld device (10) for measuring the static water pressure is used to automatically measure the static water pressure again which is upstream of the valve and is therefore in the water line to which the water intake point is connected.

5. Method according to one or more of the preceding claims, **characterized in that**, after the measurements which are carried out using the mobile handheld device (10) have been completed, one or more results of the measurements are automatically displayed on a display unit (28) of the mobile handheld device (10), and/or **in that** one or more results of the measurements performed are automatically transmitted to a remote receiving unit.

## Revendications

1. Procédé d'exploitation d'un appareil portatif mobile (10) permettant de mesurer des paramètres d'eau d'une eau coulant depuis un point de prélèvement d'eau, en particulier de l'eau potable, qui est branché sur une évacuation d'eau (24) d'un point de prélèvement d'eau pour effectuer des mesures de paramètres d'eau physiques et/ou chimiques et/ou biologiques, l'appareil portatif mobile (10) disposant de plusieurs dispositifs de mesure pour mesurer les paramètres d'eau, dans lequel on mesure avec un dispositif de mesure, notamment un compteur d'eau, dans le cadre d'une mesure de quantité d'eau, la quantité ou le volume de l'eau coulant depuis le début de la mesure de quantité d'eau du point de prélèvement d'eau à travers l'appareil portatif mobile (10), une vanne de l'appareil portatif mobile (10) qui permet de bloquer le débit de l'eau à travers l'appareil portatif mobile (10) étant fermée automatiquement si la mesure de quantité d'eau indique que la quantité d'eau qui a coulé à travers l'appareil portatif mobile (10) depuis le début de la mesure correspond à une condition prédéterminée, dans lequel, au moyen d'un dispositif de mesure de l'appareil portatif mobile (10) pour mesurer la température de l'eau après l'opération de fermeture de la vanne, la température de l'eau provenant du point de prélèvement d'eau est mesurée automatiquement, dans lequel, après l'opération de fermeture de la vanne et après que de l'eau à température élevée est sortie du point de prélèvement d'eau, la vanne est à nouveau ouverte et la température de l'eau coulant depuis le point de prélèvement d'eau est mesurée à plusieurs reprises, en particulier en continu, au moyen du dispositif de mesure de l'appareil portatif (10) pour mesurer la température de l'eau, et dans lequel la vanne est à nouveau fermée automatiquement si les mesures indiquent que la température de l'eau correspond à des critères prédéterminés, en particulier qu'elle est stable d'une manière prédéterminée.

2. Procédé selon la revendication 1, **caractérisé en ce qu'**après l'opération de fermeture de la vanne, au moyen d'un dispositif de mesure de l'appareil portatif mobile (10) pour mesurer la pression d'eau statique, la pression d'eau statique, qui est présente en amont de la vanne et donc dans la conduite d'eau sur laquelle est branché le point de prélèvement d'eau, est mesurée automatiquement.

3. Procédé selon une ou plusieurs des autres revendications, **caractérisé en ce que** l'ouverture de la vanne après l'opération de fermeture de la vanne, et après que de l'eau à température élevée a coulé du point de prélèvement d'eau, est déclenchée par un actionnement d'un dispositif d'entrée (28) de l'appareil portatif mobile (10) par un opérateur, le dispositif d'entrée étant en relation active avec un dispositif de commande électronique commandant la vanne.

4. Procédé selon la revendication 3 lorsqu'elle dépend de la revendication 2, **caractérisé en ce qu'**après la nouvelle opération de fermeture de la vanne, la pression d'eau statique, qui est présente en amont de la vanne et donc dans la conduite d'eau sur laquelle est branché le point de prélèvement d'eau, est à nouveau mesurée automatiquement au moyen du dispositif de mesure de l'appareil portatif mobile (10) pour mesurer la pression d'eau statique.

5. Procédé selon une ou plusieurs des revendications précédentes, **caractérisé en ce qu'**après achèvement des mesures qui sont effectuées par l'appareil portatif mobile (10), automatiquement un ou plusieurs résultats des mesures sont affichés sur une unité d'affichage (28) de l'appareil portatif mobile (10), et/ou **en ce qu'**automatiquement un ou plusieurs résultats des mesures effectuées sont envoyés à une unité de réception à distance.
